# EUROPEAN PATENT APPLICATION

(11) **EP 4 201 933 A1**
(43) Date of publication of application: **28.06.2023**
(21) Application number: 21217321.5
(22) Date of filing: 23.12.2021
(51) Int. Cl.: C07D 413/14

(54) **CRYSTALLISATION PROCESS FOR RIVAROXABAN**

(71) Applicant: Zaklady Farmaceutyczne Polpharma S.A., 83-200 Starogard Gdanski (PL)
(72) Inventor: SZULZ, Marcin, 83-200 Starogard Gdanski (PL); SZRAMKA, Roman, 83-140 Gniew (PL)
(74) Representative: Elkington and Fife LLP

(57) **Abstract**

This invention describes a process for the preparation of rivaroxaban modification I comprising: (i) recrystallising rivaroxaban in acetic acid in the presence of a reducing agent; and (ii) collecting the resultant rivaroxaban modification I.

## Description

The present invention relates to a crystallisation process, and more specifically to the preparation of rivaroxaban crystalline modification I.

Rivaroxaban is named (*S*)-5-chloro-*N*-{[2-oxo-3-[4-(3-oxomorpholin-4-yl)phenyl]-1,3-oxazolidin-5-yl]methyl}-2-thiophene-carboxamide and has the following structure:

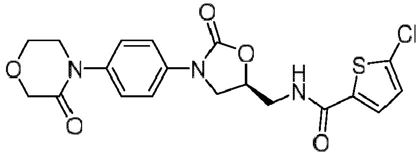

Rivaroxaban is a direct factor Xa inhibitor ("xaban"). It acts directly upon Factor X in the coagulation cascade and hence may be used as an anticoagulant. It is marketed in a number of countries as Xarelto^{®}, as an oral anticoagulant. It has been indicated for the treatment of various thromboembolic diseases, see, for example, WO 01/47949 (the basic patent), WO 2004/060887 and WO 2007/039132.

Rivaroxaban may be prepared according to the procedures described in WO 01/47949 and WO 2004/060887. Rivaroxaban is obtained in WO 01/47949 as crystalline modification I by purifying the crude product using column chromatography with a dichloromethane/methanol eluent. It is further discussed in WO 2007/039132.

WO2011/012321 discloses a purification of rivaroxaban crystalline modification I that does not require the use of column chromatography, and instead employees a recrystallisation step, wherein the recrystallisation solvent is acetic acid. CA2553237 discloses a similar purification step albeit on a much larger scale, where 2 kilograms of solvent-containing crude rivaroxaban are recrystallised from acetic acid at 100-115°C.

Modification I has a melting point of approximately 230°C and a characteristic DSC, X-ray powder diffractogram, IR spectrum, Raman spectrum, FIR spectrum and NIR spectrum as set out in Figs 1-6 of WO 2007/039132.

The characterising peaks are reported as follows.

XRPD 2θ (°): 8.9, 12.0, 14.3, 16.5, 17.4, 18.1, 19.5, 19.9, 21.7, 22.5, 23.4, 24.1, 24.5, 24.7, 25.6, 26.4, 26.7, 30.0, 30.1 and 31.8.

IR (cm⁻¹): 564, 686, 708, 746, 757, 830, 846, 920, 991, 1011, 1056, 1077, 1120, 1146, 1163, 1219, 1286, 1307, 1323, 1341, 1374, 1411, 1429, 1470, 1486, 1517, 1546, 1605, 1646, 1669, 1737, 2867, 2895, 2936, 2976 and 3354.

Raman (cm⁻¹): 84, 111, 642, 672, 687, 745, 779, 792, 1083, 1099, 1232, 1280, 1307, 1325, 1343, 1428, 1473, 1485, 1548, 1605, 1638, 1664, 1722, 2899, 2944, 2983 and 3074.

FIR (cm⁻¹): 82, 97, 138, 169, 179, 210, 226, 247, 272, 283, 298, 303, 350, 394, 417, 438, 458, 475 and 484.

NIR (cm⁻¹): 4082, 4142, 4170, 4228, 4299, 4376, 4429, 4479, 4633, 4791, 4877, 4907, 5081, 5760, 5885, 6002, 6441, 6564, 8473 and 8833.

A drawback of the procedures set out in WO2011/012321 and CA2553237 is that although rivaroxaban crystalline modification I is obtained without the requirement for column chromatography, it is obtained with sub-optimal purity.

Therefore, there remains a need in the art for an improved process for the preparation of modification I.

Accordingly, the present invention provides a process for the preparation of rivaroxaban modification I comprising:
(i) recrystallising rivaroxaban in acetic acid in the presence of a reducing agent; and
(ii) collecting the resultant rivaroxaban modification I.

It has been found that this process provides rivaroxaban modification I in high yield and high purity without recourse to column chromatography. It also provides rivaroxaban modification I with an improved purity level compared to the process in which only acetic acid is used for the recrystallisation step.

It is thought that the addition of the reducing agent to the mixture of acetic acid and rivaroxaban prevents any oxidation of rivaroxaban by the acetic acid or by any contaminant present within the acetic acid. This advantageously reduces the likelihood that oxidised rivaroxaban by products are formed during the recrystallisation process (that cannot be removed by recrystallisation), and in turn increases the purity of the recrystallised rivaroxaban modification I product.

The present invention will now be described with reference to the accompanying drawings, in which:
Fig. 1 shows the XRPD spectrum for rivaroxaban modification I;
Fig. 2 shows the DSC traces for rivaroxaban modification I;
Fig. 3 shows the IR spectrum for rivaroxaban modification I; and

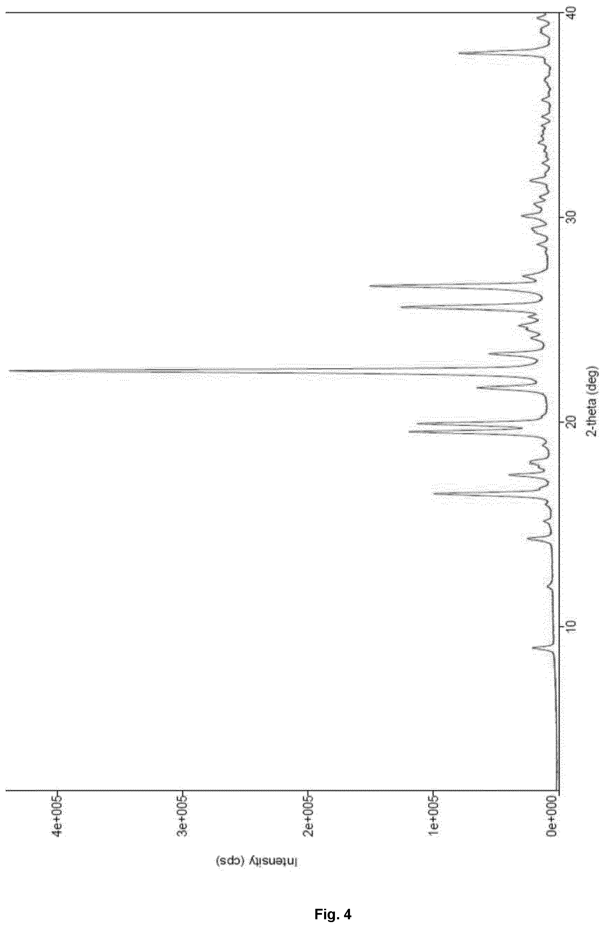

Fig. 4 shows the XRPD spectrum for rivaroxaban modification I crystallised from acetic acid.

In step (i) of the process, crude rivaroxaban is combined with acetic acid and a reducing agent. The mixture is then heated, typically to a temperature below the boiling point of acetic acid, (i.e. below 118°C) in order to effect complete dissolution of rivaroxaban and so initiate the recrystallisation process. After heating, the mixture is filtered and the filtrate is cooled to allow crystallisation to proceed.

The acetic acid used in the process is preferably glacial acetic acid, which is also known as water-free or anhydrous acetic acid. Alternatively, the glacial acetic may contain water, for example between 0.5 and 10% water by weight.

In one embodiment, the reducing agent is an alkali metal-containing reducing agent, a reducing sugar or a reducing acid agent.

An alkali metal-containing reducing agent is a reducing agent that contains a metallic element from Group 1 of the Periodic Table. Typically, the alkali metal-containing reducing agent will contain a metallic element selected from lithium, sodium or potassium, preferably the alkali metal-containing reducing agent will contain sodium.

In a preferred embodiment, the reducing agent will contain sodium, and for example be selected from sodium dithionate (Na₂S₂O₄), sodium sulfite (Na₂SO₃), sodium bisulfite (NaHSO₃), sodium sulfide (Na₂S), sodium borohydride (NaBH₄), sodium cyanoborohydride (NaBH₃CN), sodium triacetoxyborohydride (C₆H₁₀BNaO₆), sodium hydroxymethanesulfinate (CH₃NaO₃S), or sodium hypophosphite (NaPO₂H₂). Most preferably the alkali metal-containing reducing agent is sodium dithionate (Na₂S₂O₄), sodium sulfite (Na₂SO₃), sodium sulfide (Na₂S) or sodium borohydride (NaBH₄).

Alternatively, the reducing agent will contain lithium or potassium, for example lithium borohydride (LiBH₄) or potassium borohydride (KBH₄). Furthermore, any combination of reducing agents may be used, for example a combination or sodium borohydride and lithium borohydride.

The reducing agent may also be a reducing sugar.

A reducing sugar is a sugar that can readily interconvert (or degrade) into a reactive aldehyde derivative, which in turn can act as a reducing agent. An example of a reducing sugar is lactose, which can interconvert to reveal a reactive aldehyde derivative as follows:

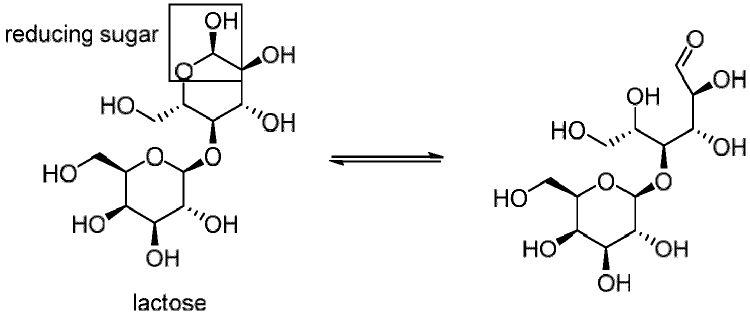

In one embodiment the reducing sugar is selected from galactose, glucose, D-(+)-glucose, dextrose, glyceraldehyde, fructose, ribose, xylose, arabinose, cellobiose, lactose, maltitol and maltose.

The reducing agent may also be a reducing acid agent.

A reducing acid agent is an acid that is capable of behaving as a reducing agent. Preferred examples of reducing acids include phosphorous acid, ascorbic acid, formic acid and oxalic acid, and salts thereof. D- and L-ascorbic acid are suitable reducing acids.

In a preferred embodiment, the reducing agent is selected from sodium dithionite (Na₂S₂O₄), sodium sulfite (Na₂SO₃), sodium sulfide (Na₂S), sodium borohydride (NaBH₄), phosphorous acid (H₃PO₃), ascorbic acid, D-(+)-glucose, dextrose and dimethyl sulfoxide.

The reducing agent may be used in an amount of from 1.0 mol% to 15 mol% relative to rivaroxaban. Preferably, the reducing agent is used in an amount of from 2.0 mol% to 11.5 mol%, or 5.0 mol% to 7.5 mol% relative to rivaroxaban.

In one embodiment, the reducing agent is selected from an alkali metal-containing reducing agent, a reducing sugar or a reducing acid agent, and is used in an amount of from 1.0 mol% to 15 mol% relative to rivaroxaban, preferably of from 2.0 mol% to 11.5 mol%, or 5.0 mol% to 7.5 mol% relative to rivaroxaban. Preferably the alkali metal-containing reducing agent contains sodium.

In a further embodiment, the reducing agent is selected from sodium dithionate (Na₂S₂O₄), sodium sulfite (Na₂SO₃), sodium sulfide (Na₂S), sodium borohydride (NaBH₄), phosphorous acid and ascorbic acid and is used in an amount of from 1.0 mol% to 15 mol% relative to rivaroxaban, preferably of from 2.0 mol% to 11.5 mol%, or 5.0 mol% to 7.5 mol% relative to rivaroxaban.

In step (i) of the process, the recrystallisation step comprises combining rivaroxaban, acetic acid and the reducing agent, and heating the resulting mixture to between 65°C and 95°C. Preferably the mixture is heated to between 70°C and 90°C, more preferably between 80°C and 85°C.

Once heated, the resulting mixture is typically stirred for a duration of between 2 and 6 hours, preferably 4 hours.

The recrystallisation step may comprise combining rivaroxaban, acetic acid and an alkali metal-containing reducing agent, a reducing sugar or a reducing acid agent, and heating the resulting mixture to between 65°C and 95°C, preferably between 70°C and 90°C, or between 80°C and 85°C. Preferably the alkali metal-containing reducing agent contains sodium.

The recrystallisation step may also comprise combining rivaroxaban, acetic acid and a reducing agent selected from sodium dithionate (Na₂S₂O₄), sodium sulfite (Na₂SO₃), sodium sulfide (Na₂S), sodium borohydride (NaBH₄), phosphorous acid and ascorbic acid, and heating the resulting mixture to between 65°C and 95°C, preferably between 70°C and 90°C, or between 80°C and 85°C.

Alternatively, the recrystallisation step may also comprise combining rivaroxaban, acetic acid and a reducing agent selected from sodium dithionite (Na₂S₂O₄), sodium sulfite (Na₂SO₃), sodium sulfide (Na₂S), sodium borohydride (NaBH₄), phosphorous acid (H₃PO₃), ascorbic acid, D-(+)-glucose, dextrose and dimethyl sulfoxide, and heating the resulting mixture to between 65°C and 95°C, preferably between 70°C and 90°C, or between 80°C and 85°C.

The recrystallisation step may comprise combining rivaroxaban, acetic acid and an alkali metal-containing reducing agent, a reducing sugar or a reducing acid agent in an amount of from 1.0 mol% to 15 mol% relative to rivaroxaban, preferably of from 2.0 mol% to 11.5 mol%, or 5.0 mol% to 7.5 mol% relative to rivaroxaban, and heating the resulting mixture to between 65°C and 95°C, preferably between 70°C and 90°C, or between 80°C and 85°C. Preferably the alkali metal-containing reducing agent contains sodium.

The recrystallisation step may also comprise combining rivaroxaban, acetic acid and a reducing agent selected from sodium dithionate (Na₂S₂O₄), sodium sulfite (Na₂SO₃), sodium sulfide (Na₂S), sodium borohydride (NaBH₄), phosphorous acid and ascorbic acid in an amount of from 1.0 mol% to 15 mol% relative to rivaroxaban, preferably of from 2.0 mol% to 11.5 mol% or 5.0 mol% to 7.5 mol% relative to rivaroxaban, and heating the resulting mixture to between 65°C and 95°C, preferably between 70°C and 90°C, or between 80°C and 85°C.

Alternatively, the recrystallisation step may comprise combining rivaroxaban, acetic acid and a reducing agent selected from sodium dithionite (Na₂S₂O₄), sodium sulfite (Na₂SO₃), sodium sulfide (Na₂S), sodium borohydride (NaBH₄), phosphorous acid (H₃PO₃), ascorbic acid, D-(+)-glucose, dextrose and dimethyl sulfoxide in an amount of from 1.0 mol% to 15 mol% relative to rivaroxaban, preferably of from 2.0 mol% to 11.5 mol% or 5.0 mol% to 7.5 mol% relative to rivaroxaban, and heating the resulting mixture to between 65°C and 95°C, preferably between 70°C and 90°C, or between 80°C and 85°C.

In step (i) of the process, after heating, the mixture is filtered and the filtrate is cooled to allow crystallisation to proceed.

Typically, the mixture is first allowed to cool to between 50°C and 70°C, preferably between 55°C and 65°C, and more preferably between 60°C and 65°C whilst being stirred. The mixture is typically kept at the cooling temperature for a duration of between 1 to 5 hours, preferably 2 to 4 hours, and more preferably 3 hours.

Following cooling to between 50°C and 70°C, preferably between 55°C and 65°C, and more preferably between 60°C and 65°C, for a duration of between 1 to 5 hours, preferably 2 to 4 hours, and more preferably 3 hours, the mixture is cooled further in a second cooling step.

Preferably in the second cooling step, the mixture is further cooled to between 10°C and 40°C, preferably between 15°C and 30°C, and more preferably between 20°C and 25°C whilst being stirred. The mixture is typically kept at the second cooling temperature for a duration of between 1 to 5 hours, preferably 2 to 4 hours, and more preferably 2 hours.

It is during the second cooling step that a crystalline precipitate of rivaroxaban modification I forms.

In step (i) of the process, in a preferred embodiment, after heating, the mixture is filtered and the filtrate is cooled to between 60°C and 65°C whilst being stirred. The mixture is then kept at between 60°C and 65°C for a duration of 2 to 4 hours, preferably 3 hours, before being further cooled to between 15°C and 30°C, preferably between 20°C and 25°C whilst being stirred. The mixture is then preferably kept at between 15°C and 30°C, preferably between 20°C and 25°C for a duration of between 2 to 4 hours, preferably 2 hours during which time a crystalline precipitate of rivaroxaban modification I forms.

Prior to the recrystallisation step, the rivaroxaban that is recrystallised according to the process of the present invention may be prepared by reacting a compound of formula (I) or a salt thereof, with a compound of formula (II):

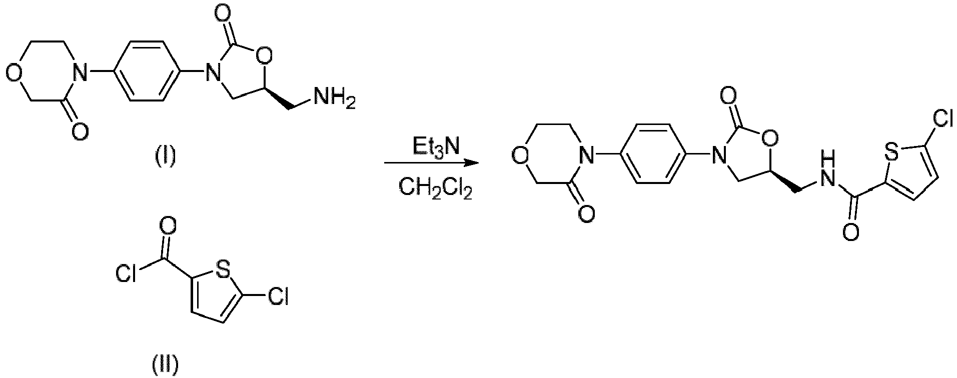

Preferably, the compound of formula (I) is used as the hydrochloride salt, and preferably the compounds of formula (I) and (II) are reacted together in the presence of trimethylamine and dichloromethane. However, other bases and solvents may also facilitate the reaction.

Prior to the recrystallisation step, the rivaroxaban prepared by reacting a compound of formula (I) or a salt thereof, with a compound of formula (II) is typically dried to remove excess solvent, including excess water.

The compound of formula (II) may be prepared by reacting a compound of formula (III) (5-chlorothiophene-2-carboxylic acid) with thionyl chloride:

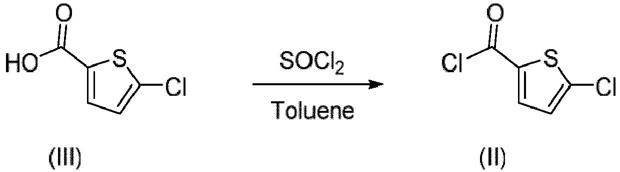

In step (ii) of the process, the resultant rivaroxaban modification I (recrystallised according to step (i) of the process) is collected.

Preferably the rivaroxaban modification I is collected by filtration of the recrystallised product from the acetic acid and the reducing agent.

Typically, the solid recrystallised product is separated by filtration, and the solid is washed with acetic acid and demineralised water before being subjected to a drying step in a vacuum drier.

The present process is straightforward and may proceed even with an impure source of rivaroxaban.

Accordingly, the process includes an embodiment where the rivaroxaban has not been subjected to column chromatography. Indeed, the rivaroxaban may be provided as a starting material for the preparation process as crude rivaroxaban having an impurity content of up to 10% by weight, e.g. 0.10-10% by weight.

In an alternative embodiment, the present invention provides a process for the preparation of rivaroxaban modification I comprising:
(i) treating acetic acid with a reducing agent;
(ii) separating the treated acetic acid from the reducing agent;
(iii) recrystallising rivaroxaban in the treated acetic acid; and
(iv) collecting the resultant rivaroxaban modification I.

It is thought that treatment of the acetic acid prior to the recrystallisation step reduces the tendency of the acetic acid or any contaminant present within the acetic acid to oxidise rivaroxaban during the recrystallisation step. This advantageously reduces the likelihood that oxidised rivaroxaban byproducts are formed during the recrystallisation process (that cannot be removed by recrystallisation), and in turn increases the purity of the recrystallised rivaroxaban modification I product.

In step (i) of the process, the acetic acid is treated with a reducing agent.

The acetic acid used in the process is typically glacial acetic acid, as defined hereinabove.

In a preferred embodiment, the reducing agent is an alkali metal-containing reducing agent, a reducing sugar or a reducing acid agent as defined hereinabove.

Typically, the alkali metal-containing reducing agent will contain a metallic element selected from lithium, sodium or potassium, preferably the alkali metal-containing reducing agent will contain sodium.

In a preferred embodiment, the reducing agent will contain sodium, and for example be selected from sodium dithionate (Na₂S₂O₄), sodium sulfite (Na₂SO₃), sodium bisulfite (NaHSO₃), sodium sulfide (Na₂S), sodium borohydride (NaBH₄), sodium cyanoborohydride (NaBH₃CN), sodium triacetoxyborohydride (C₆H₁₀BNaO₆), sodium hydroxymethanesulfinate (CH₃NaO₃S), or sodium hypophosphite (NaPO₂H₂). Most preferably the alkali metal-containing reducing agent is sodium dithionate (Na₂S₂O₄), sodium sulfite (Na₂SO₃), sodium sulfide (Na₂S) or sodium borohydride (NaBH₄).

Alternatively, the reducing agent will contain lithium or potassium, for example lithium borohydride (LiBH₄) or potassium borohydride (KBH₄). Furthermore, any combination of reducing agents may be used, for example a combination or sodium borohydride and lithium borohydride

In one embodiment, the reducing sugar is selected from galactose, glucose, D-(+)-glucose, dextrose, glyceraldehyde, fructose, ribose, xylose, arabinose, cellobiose, lactose, maltitol and maltose.

In one embodiment, the reducing acid is selected from phosphorous acid, ascorbic acid, formic acid and oxalic acid and salts thereof. D- and L-ascorbic acid are suitable reducing acids.

In a preferred embodiment, the reducing agent is selected from sodium dithionite (Na₂S₂O₄), sodium sulfite (Na₂SO₃), sodium sulfide (Na₂S), sodium borohydride (NaBH₄), phosphorous acid (H₃PO₃), ascorbic acid, D-(+)-glucose, dextrose and dimethyl sulfoxide.

In step (i) of the process, the treatment of acetic acid comprises combining acetic acid and the reducing agent, and heating the resulting mixture to between 50°C and 80°C. Preferably the mixture is heated to between 60°C and 70°C.

Once heated, the resulting mixture is typically stirred for a duration of between 1 and 4 hours, preferably 2 hours.

Step (i) of the process may comprise combining acetic acid and an alkali metal-containing reducing agent, a reducing sugar or a reducing acid agent, and heating the resulting mixture to between 50°C and 80°C, preferably between 60°C and 70°C. Preferably the alkali metal-containing reducing agent contains sodium.

Step (i) of the process may comprise combining acetic acid and a reducing agent selected from sodium dithionate (Na₂S₂O₄), sodium sulfite (Na₂SO₃), sodium borohydride (NaBH₄), phosphorous acid and ascorbic acid, and heating the resulting mixture to between 50°C and 80°C, preferably between 60°C and 70°C

Step (i) of the process may also comprise combining acetic acid and a reducing agent selected from sodium dithionite (Na₂S₂O₄), sodium sulfite (Na₂SO₃), sodium sulfide (Na₂S), sodium borohydride (NaBH₄), phosphorous acid (H₃PO₃), ascorbic acid, D-(+)-glucose, dextrose and dimethyl sulfoxide, and heating the resulting mixture to between 50°C and 80°C, preferably between 60°C and 70°C

In step (ii) of the process, the treated acetic acid is separated from the reducing agent, for example by filtration.

In step (iii) of the process, the treated acetic acid is combined with rivaroxaban, and the rivaroxaban is recrystallised from the treated acetic acid.

In step (iii) of the process, the crude rivaroxaban and the treated acetic acid are combined, and the mixture is then heated, typically to a temperature below the boiling point of acetic acid, (i.e. below 118°C) in order to effect complete dissolution of rivaroxaban and so initiate the recrystallisation process. After heating, the mixture is filtered and the filtrate is cooled to allow crystallisation to proceed.

Typically, the recrystallisation step comprises combining rivaroxaban and the treated acetic acid, and heating the resulting mixture to between 65°C and 95°C. Preferably the mixture is heated to between 70°C and 90°C, more preferably between 80°C and 85°C.

Once heated, the resulting mixture is typically stirred for a duration of between 2 and 6 hours, preferably 4 hours.

In step (iii) of the process, after heating, the mixture is filtered and the filtrate is cooled to allow crystallisation to proceed.

Typically the mixture is first allowed to cool to between 50°C and 70°C, preferably between 55°C and 65°C, and more preferably between 60°C and 65°C whilst being stirred. The mixture is typically kept at the cooling temperature for a duration of between 1 to 5 hours, preferably 2 to 4 hours, and more preferably 3 hours.

Following cooling to between 50°C and 70°C, preferably between 55°C and 65°C, and more preferably between 60°C and 65°C, for a duration of between 1 to 5 hours, preferably 2 to 4 hours, and more preferably 3 hours, the mixture is cooled further in a second cooling step.

Preferably in the second cooling step, the mixture is further cooled to between 10°C and 40°C, preferably between 15°C and 30°C, and more preferably between 20°C and 25°C whilst being stirred. The mixture is typically kept at the second cooling temperature for a duration of between 1 to 5 hours, preferably 2 to 4 hours, and more preferably 2 hours.

It is during the second cooling step that a crystalline precipitate of rivaroxaban modification I forms.

In step (iii) of the process, in a preferred embodiment, after heating, the mixture is filtered and the filtrate is cooled to between 60°C and 65°C whilst being stirred. The mixture is then kept at between 60°C and 65°C for a duration of 2 to 4 hours, preferably 3 hours, before being further cooled to between 15°C and 30°C, preferably between 20°C and 25°C whilst being stirred. The mixture is then preferably kept at between 15°C and 30°C, preferably between 20°C and 25°C for a duration of between 2 to 4 hours, preferably 2 hours during which time a crystalline precipitate of rivaroxaban modification I forms.

Prior to the recrystallisation step, the rivaroxaban that is combined with the treated acetic acid and recrystallised may be prepared according to the process defined hereinabove.

In step (iv) of the process, the resultant rivaroxaban modification I (recrystallised according to step (iii) of the process) is collected.

Preferably the rivaroxaban modification I is collected by filtration of the recrystallised product from the treated acetic acid.

Typically, the solid recrystallised product is separated by filtration, and the solid is washed with treated acetic acid and demineralised water before being subjected to a drying step in a vacuum drier.

The present process is straightforward and may proceed even with an impure source of rivaroxaban.

Accordingly, the process includes an embodiment where the rivaroxaban has not been subjected to column chromatography. Indeed, the rivaroxaban may be provided as a starting material for the preparation process as crude rivaroxaban having an impurity content of up to 10% by weight, e.g. 0.10-10% by weight.

Rivaroxaban is used for the treatment of thromboembolic diseases. The thromboembolic diseases are defined in more detail in WO 2007/039132. They include myocardial infarction with ST segment elevation (STEMI) and without ST segment elevation (non-STEMI), stable angina pectoris, unstable angina pectoris, reocclusions and restenoses after coronary interventions such as angioplasty or aortocoronary bypass, peripheral arterial occlusive diseases, pulmonary embolisms, deep vein thromboses and renal vein thromboses, transitory ischemic attacks, thrombotic and thromboembolic cerebral stroke, cerebral ischemias, stroke and systemic thromboembolisms and ischemias in patients with acute, intermittent or persistent cardiac arrhythmias, for example, atrial fibrillation, and those who are subject to cardioversion, furthermore in the case of patients with heart valve diseases or with artificial heart valves, disseminated intravasal clotting (DIC), microangiopathic hemolytic anemias, extracorporeal blood circulations, for example hemodialysis, and heart valve prostheses, atherosclerotic vascular diseases and inflammatory diseases such as rheumatic diseases of the locomotor system, Alzheimer's disease, inhibition of tumour growth and of metastasis formation, in microangiopathies, age-related macular degeneration, diabetic retinopathy, diabetic nephropathy and other microvascular diseases, and for the prevention and treatment of thromboembolic complications, for example, venous thromboembolisms, in tumour patients, in particular those who are subjected to relatively large surgical interventions or chemo/radiotherapy, the prevention of coagulation ex vivo, e.g. for the preservation of blood and plasma products, for the cleaning/pretreatment of catheters and other medical aids and equipment, for the coating of artificial surfaces of medical aids and equipment employed in vivo or ex vivo or in biological samples which contain factor Xa. It may also be used for the prevention of blood coagulation in vitro, in particular in blood preserves or biological samples which contain factor Xa.

Accordingly, the present invention also includes a process for preparing a rivaroxaban dosage form, comprising the steps of preparing rivaroxaban modification I as described herein and combining the rivaroxaban modification I with one or more pharmaceutically acceptable excipients. Rivaroxaban is typically administered orally and so the dosage form is preferably an oral dosage form, most preferably a tablet. In such a case, the process further comprises a tableting step. The oral dosage form may be a coated or uncoated tablet and may be prepared using standard techniques known in the art.

In the case of oral administration, the dose is usually 0.01 to 100 mg/Kg, preferably 0.01 to 20 mg/Kg and most preferably 0.1 to 10 mg/kg.

The present invention will now be described with reference to the following examples, which are not intended to be limiting.

### Examples

### General methods

A number of experiments were performed in which crude rivaroxaban was recrystallised from acetic acid in the presence of a reducing agent to provide crystalline rivaroxaban modification I.

A number of comparative experiments were also performed in which rivaroxaban was recrystallised from acetic acid in the absence of a reducing agent to provide crystalline rivaroxaban modification I.

Rivaroxaban modification I was characterised as follows.

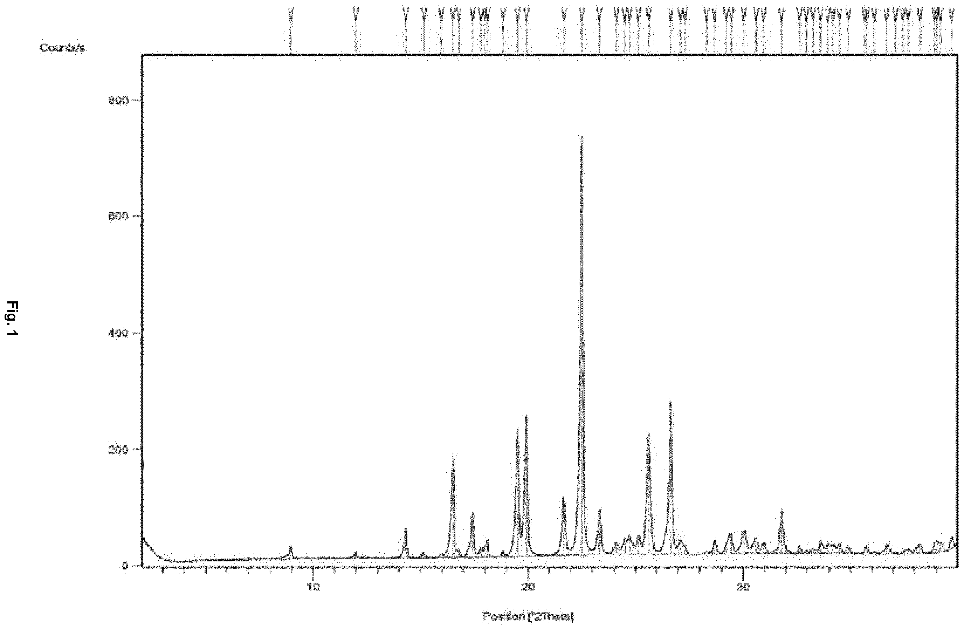

The sample was ground and XRPD was performed. The spectrum is shown in Fig. 1. The XRPD spectra were obtained using a PANalytical X'Pert PRO MPD diffractometer in Bragg-Brentano geometry, X'Celerator RTMS Detector. The apparatus used Cu K-alpha radiation (0.15418740 nm) with a scan range of 1.9990-40.0004° and a step size of 0.0167°.

The characterising diffraction lines are at 2θ (°): 16.5, 19.5, 22.5 and 23.4.

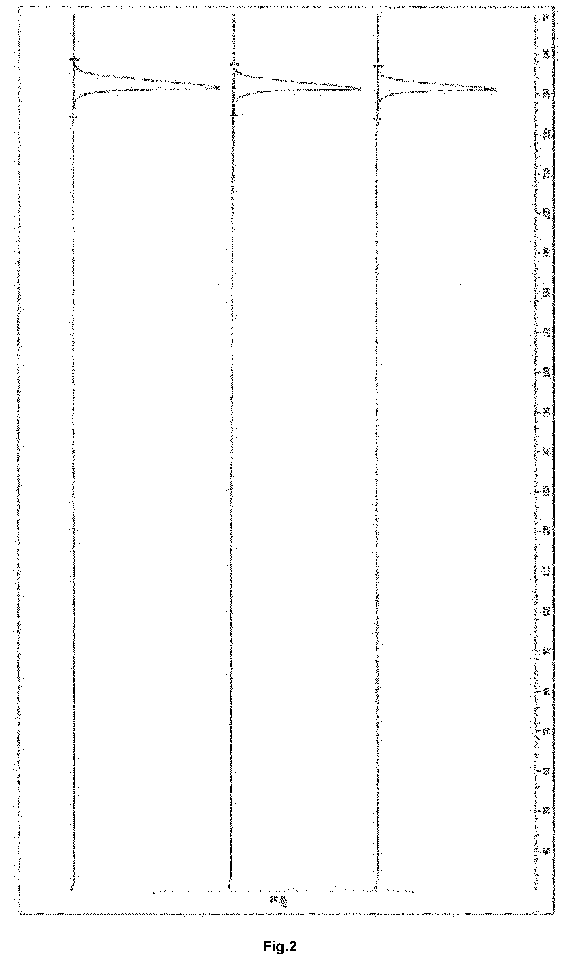

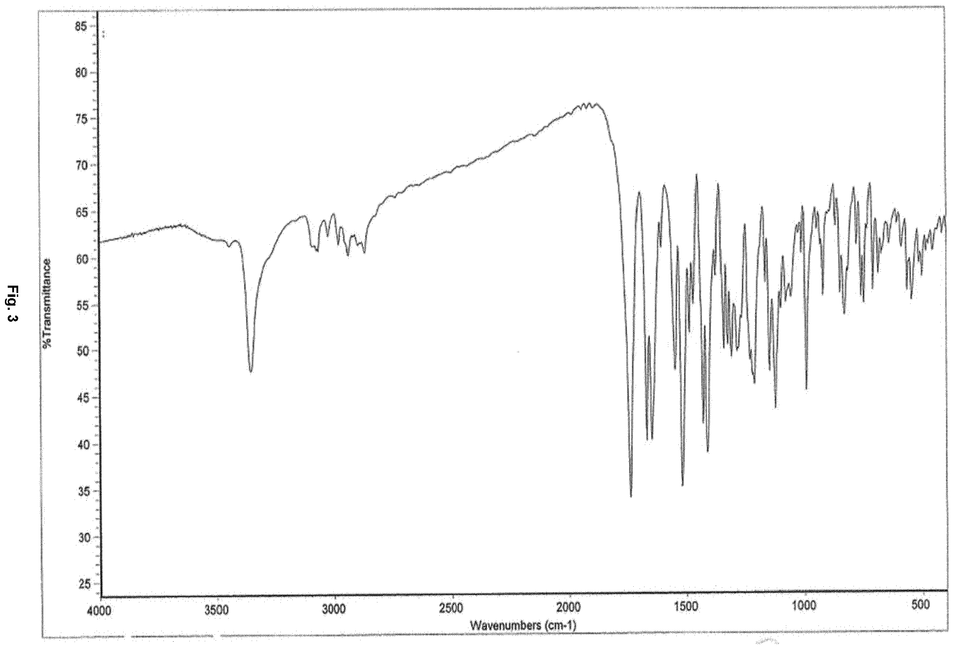

Differential scanning calorimetry (DSC) and infrared spectroscopy were also used and the DSC trace and IR spectrum are shown in Figs 2 and 3, respectively.

DSC was performed using a DSC822e Mettler-Toledo apparatus at a heating rate of 10.0°C/min.

The IR spectra were obtained according to Ph.Eur. (2.2.24.) and USP, <197K> using the KBr disc method. The apparatus was Avatar 370 FT-IR Thermo-Nicolet. The IR peak listing are at (cm⁻¹): 416, 455, 483, 501, 564, 587, 607, 640, 671, 685, 707, 731, 745, 756, 775, 813, 828, 846, 864, 920, 930, 946, 991, 1011, 1023, 1055,1076, 1098, 1120, 1145, 1163, 1210, 1219, 1285, 1307, 1323, 1340, 1373, 1409, 1429, 1469, 1486, 1517, 1546, 1605, 1646, 1669, 1737, 2867, 2936, 2976, 3023, 3066, 3354 and 3442. The characteristic peaks are at (cm⁻¹): 564, 685, 707, 745, 756, 828, 846, 920, 991, 1011, 1055, 1076, 1120, 1145, 1163, 1219, 1285, 1307, 1323, 1340, 1373, 1409, 1429, 1469, 1486, 1517, 1546, 1605, 1646, 1669, 1737, 2867, 2936, 2976 and 3354.

Raman spectroscopy was also performed. The spectra were obtained using a Thermo Nicolet Almega XR. Spectra were consistent with modification I.

The purity of the rivaroxaban modification I obtained was assessed by HPLC according to the following protocol.

Solution A: Dissolve 1.36 g of potassium dihydrogen phosphate in water for chromatography R, add 200 µL of phosphoric acid and dilute to 1000 mL with water for chromatography.

Solvent mixture: acetonitrile, solution A (40:60 V/V).

Test solution: Dissolve 25.0 mg of the substance to be examined in the solvent mixture and dilute to 50.0 mL with the solvent mixture.

Reference solution (a): Dissolve 25.0 mg of rivaroxaban CRS in the solvent mixture and dilute to 50.0 mL with the solvent mixture.

Reference solution (b): Dilute 1.0 mL of the test solution to 100.0 mL with the solvent mixture. Dilute 1.0 mL of this solution to 10.0 mL with the solvent mixture.

Reference solution (c): Dissolve 2.5 mg of rivaroxaban for system suitability CRS (containing impurity G) in the solvent mixture and dilute to 5 mL with the solvent mixture.
Column:
   - size: I =0.15 m, Ø=3.0mm;
   - stationary phase: end-capped extra-dense bonded octadecylsilyl silica gel for chromatography R (3.5m) (ZORBAX Eclipse XDB C18 is suitable.);
   - temperature:60°C.
Mobile phase:
   - mobile phase A: mix 5 volumes of methanol and 95 volumes of a 1.0 g/L solution of sodium hexanesulfonate in solution A;
   - mobile phase B: acetonitrile;
Flow rate: 1.0 mL/min.
Detection: spectrophotometer at 250 nm.
Injection: 3 µL of the test solution and reference solutions (b) and (c).

### Synthesis of compound (III)

5-chlorothiophene-2-carboxylic acid (24.39 g) and thionyl chloride (43.5 mL, 71.39 g) were added to a reactor, and the reactor was heated to 50-60°C for 1 hour. The reaction mixture was maintained for 4 hours at 50-60°C. Upon completion of the reaction excess thionyl chloride was distilled out at 50-60°C under vacuum (∼60 mBar). Toluene (50 mL, 43.1 g) was then added to the reactor and the remaining residue of thionyl chloride was distilled out under vacuum (∼60 mBar). The process of adding toluene to the remaining residue and distillation was repeated a further two times.

Following this, the remaining residue in the reactor (compound (III)) was used without further purification in the next synthetic step.

### Synthesis of crude rivaroxaban

Triethylamine (48.5 mL, 35.09 g), dichloromethane (225.62 mL, 300.0 g) and 4-[4-[(5S)-5-(aminomethyl)-2-oxazolidin-3-yl]phenyl]-3-morpholinone hydrochloride (44.63 g) were added to a reactor, and the resulting mixture was stirred for 1 hour at 20-25°C. Compound (III) was then added dropwise to the reactor at 20-30°C. The resulting mixture was then heated to 38-40°C for 1 hour and maintained at 38-40°C for a further hour. The mixture was then cooled to 20-25°C, and then water (446 mL) was added. The resulting mixture was then mixed at 10-25°C for 30 minutes.

Following this, a solid was observed and obtained via filtration under reduced pressure. The resulting filter cake was washed with water (3 x 60 mL), before the obtained solid was dried under reduced pressure at 65-70°C to provide crude rivaroxaban in 85-95% yield and about 98% purity.

### Example 1: Recrvstallisation of crude rivaroxaban from acetic acid and sodium dithionite (Na₂S₂O₄)

A reactor was loaded with 150 mL (156.69 g, 2.61 mol) of acetic acid, 15 g (0.035 mol) of crude rivaroxaban, 0.15 g (0.001 mol) of sodium dithionite and 0.45 g (0.038 mol) of activated carbon, and heated to 85°C. The reactor was maintained for 4 hours at 80-85°C with stirring. The resulting mixture was then filtered into a second reactor, and the filter plate was washed with 10 mL of acetic acid. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid, followed by five 200 mL portions of demineralised water. Following the washing step, the precipitate was dried in a vacuum drier for 20 hours at 70°C, to provide 12.5 g of a white crystalline powder of rivaroxaban modification I in a yield of 83.3%.

### Example 2: Recrvstallisation of crude rivaroxaban from acetic acid and sodium sulfite (Na₂SO₃)

A reactor was loaded with 150 mL (156.69 g, 2.61 mol) of acetic acid, 15 g (0.035 mol) of crude rivaroxaban and 0.15 g (0.001 mol) of sodium sulfite, and heated to 85°C. The reactor was maintained for 4 hours at 80-85°C with stirring. The resulting mixture was then filtered into a second reactor, and the filter plate was washed with 10 mL of acetic acid. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid, followed by five 200 mL portions of demineralised water. Following the washing step, the precipitate was dried in a vacuum drier for 20 hours at 70°C, to provide 12.3 g of a white crystalline powder of rivaroxaban modification I in a yield of 82.0%.

### Example 3: Recrvstallisation of crude rivaroxaban from acetic acid and sodium sulfide (Na₂S)

A reactor was loaded with 150 mL (156.69 g, 2.61 mol) of acetic acid, 15 g (0.035 mol) of crude rivaroxaban and 0.15 g (0.002 mol) of sodium sulfide, and heated to 85°C. The reactor was maintained for 4 hours at 80-85°C with stirring. The resulting mixture was then filtered into a second reactor, and the filter plate was washed with 10 mL of acetic acid. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid, followed by five 200 mL portions of demineralised water. Following the washing step, the precipitate was dried in a vacuum drier for 20 hours at 70°C, to provide 12.5 g of a light beige crystalline powder of rivaroxaban modification I in a yield of 83.3%.

### Example 4: Recrvstallisation of crude rivaroxaban from acetic acid and sodium borohvdride (NaBH₄)

A reactor was loaded with 150 mL (156.69 g, 2.61 mol) of acetic acid, 15 g (0.035 mol) of crude rivaroxaban and 0.15 g (0.004 mol) of sodium borohydride, and heated to 85°C. The reactor was maintained for 4 hours at 80-85°C with stirring. The resulting mixture was then filtered into a second reactor, and the filter plate was washed with 10 mL of acetic acid. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid, followed by five 200 mL portions of demineralised water. Following the washing step, the precipitate was dried in a vacuum drier for 20 hours at 70°C, to provide 13.1 g of a light yellow crystalline powder of rivaroxaban modification I in a yield of 87.3%.

### Example 5: Recrvstallisation of crude rivaroxaban from acetic acid and phosphorous acid (H₃PO₃)

A reactor was loaded with 150 mL (156.69 g, 2.61 mol) of acetic acid, 15 g (0.035 mol) of crude rivaroxaban and 0.15 g (0.002 mol) of phosphorous acid, and heated to 85°C. The reactor was maintained for 4 hours at 80-85°C with stirring. The resulting mixture was then filtered into a second reactor, and the filter plate was washed with 10 mL of acetic acid. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid, followed by five 200 mL portions of demineralised water. Following the washing step, the precipitate was dried in a vacuum drier for 20 hours at 70°C, to provide 12.6 g of a white crystalline powder of rivaroxaban modification I in a yield of 83.7%.

### Example 6: Recrvstallisation of crude rivaroxaban from acetic acid and ascorbic acid

A reactor was loaded with 150 mL (156.69 g, 2.61 mol) of acetic acid, 15 g (0.035 mol) of crude rivaroxaban and 0.15 g (0.001 mol) of L-ascorbic acid, and heated to 85°C. The reactor was maintained for 4 hours at 80-85°C with stirring. The resulting mixture was then filtered into a second reactor, and the filter plate was washed with 10 mL of acetic acid. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid, followed by five 200 mL portions of demineralised water. Following the washing step, the precipitate was dried in a vacuum drier for 20 hours at 70°C, to provide 12.7 g of a white crystalline powder of rivaroxaban modification I in a yield of 84.7%.

### Example 7: Recrvstallisation of crude rivaroxaban from acetic acid and D-(+)-glucose

A reactor was loaded with 150 mL (156.69 g, 2.61 mol) of acetic acid, 15 g (0.035 mol) of crude rivaroxaban and 0.15 g (0.001 mol) of D-(+)-glucose, and heated to 85°C. The reactor was maintained for 4 hours at 80-85°C with stirring. The resulting mixture was then filtered into a second reactor, and the filter plate was washed with 10 mL of acetic acid. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid, followed by five 200 mL portions of demineralised water. Following the washing step, the precipitate was dried in a vacuum drier for 20 hours at 70°C, to provide 12.5 g of a beige crystalline powder of rivaroxaban modification I in a yield of 83.3%.

### Example 8: Recrvstallisation of crude rivaroxaban from acetic acid and dextrose

A reactor was loaded with 150 mL (156.69 g, 2.61 mol) of acetic acid, 15 g (0.035 mol) of crude rivaroxaban and 0.15 g (0.001 mol) of dextrose, and heated to 85°C. The reactor was maintained for 4 hours at 80-85°C with stirring. The resulting mixture was then filtered into a second reactor, and the filter plate was washed with 10 mL of acetic acid. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid, followed by five 200 mL portions of demineralised water. Following the washing step, the precipitate was dried in a vacuum drier for 20 hours at 70°C, to provide 11.8 g of a beige crystalline powder of rivaroxaban modification I in a yield of 78.7%.

### Example 9: Recrvstallisation of crude rivaroxaban from acetic acid and dimethyl sulfoxide

A reactor was loaded with 150 mL (156.69 g, 2.61 mol) of acetic acid, 15 g (0.035 mol) of crude rivaroxaban and 0.15 g (0.002 mol) of dimethyl sulfoxide, and heated to 85°C. The reactor was maintained for 4 hours at 80-85°C with stirring. The resulting mixture was then filtered into a second reactor, and the filter plate was washed with 10 mL of acetic acid. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid, followed by five 200 mL portions of demineralised water. Following the washing step, the precipitate was dried in a vacuum drier for 20 hours at 70°C, to provide 13.0 g of a white crystalline powder of rivaroxaban modification I in a yield of 86.7%.

### Example 10: Recrystallisation of crude rivaroxaban from acetic acid (acetic acid pre-treated with reducing agent)

A reactor was loaded with 170 mL (177.58 g, 2.95 mol) of acetic acid and 0.17 g (0.0011 mol) of sodium dithionate, and heated to 85°C. The reactor was kept and said temperature for 2 hours. The resulting mixture was then filtered into a second reactor. To the filtered acetic acid (150 mL) 15 g (0.035 mol) of crude rivaroxaban was added. The reactor has heated to 85°C. The reactor was maintained for 4 hours at 80-85°C with stirring. The mixture was then cooled to 62°C, and then mixed for 3 hours at 62-65°C with stirring. The mixture was then cooled to 25°C, and then mixed for 2 hours at 25°C with stirring, during which time a crystalline precipitate was observed. The precipitate was separated by filtration, and washed with 10 mL of acetic acid (previously obtained from the filtrate of the acetic acid/dithionate mixture), followed by five 200 mL portions of demineralised water. Following the washing step, the precipitate was dried in a vacuum drier for 20 hours at 70°C, to provide 12.8 g of a white crystalline powder of rivaroxaban modification I in a yield of 85.3%.

In each case (Examples 1-10) the purity of the rivaroxaban modification I obtained was assessed by HPLC. The results are set out in Table 1.

### Reference Examples

Reference Example 1: Recrvstallisation of crude rivaroxaban from acetic acid according to the process described in Example 26 of WO2011/012321

Example 26 of WO2011/012321 reads:
The mixture of 1 g rivaroxaban and 20 mL of acetic acid was heated to obtain clear solution. The hot solution was slowly poured into 30 mL of water. The obtained suspension was stirred at room temperature for 3 hours. The precipitate was filtered, washed with water and dried at 40 to 50°C under reduced pressure.

Reference example 1 was performed according to this process, but 10 g of crude rivaroxaban were used, and the amounts of the other reagents adjusted accordingly. This provided a white crystalline powder of rivaroxaban modification I in a yield of 93.1%.

Reference Example 2: Recrvstallisation of crude rivaroxaban from acetic acid according to the process described in CA2553237

The relevant process step in CA2553237 reads:
"3rd step: 5-Chloro-N-({(5S)-2-oxo-3-[4-(3-oxo-4-morpholinyl)phenyl)-1,3- oxazolidin-5-yl}- methyl)-2-thiophenecarboxamide (I) - recrystallization"

2120 g of solvent-containing crude product (residual moisture content 9.4%) is suspended in 12 kg of acetic acid and heated to 110 to 115°C. The resulting solution is stirred at this temperature for 10 minutes and then, after clarifying filtration, cooled to 20°C. The precipitated product is filtered off with suction, washed with acetic acid and water and then dried.

Reference example 2 was performed according to this process, but 30 g of crude rivaroxaban (residual moisture content 10%) were used, and the amounts of the other reagents adjusted accordingly. This provided a light pink/grey crystalline powder of rivaroxaban modification I in a yield of 94.2%.

Reference Example 3: Recrvstallisation of crude rivaroxaban from acetic acid according to the process described in CA2553237 (time of mixing prolonged to 2 hours)

The relevant process step in CA2553237 reads:
"3rd step: 5-Chloro-N-({(5S)-2-oxo-3-[4-(3-oxo-4-morpholinyl)phenyl)-1,3- oxazolidin-5-yl}- methyl)-2-thiophenecarboxamide (I) - recrystallization"

2120 g of solvent-containing crude product (residual moisture content 9.4%) is suspended in 12 kg of acetic acid and heated to 110 to 115°C. The resulting solution is stirred at this temperature for 10 minutes and then, after clarifying filtration, cooled to 20°C. The precipitated product is filtered off with suction, washed with acetic acid and water and then dried.

Reference example 3 was performed according to this process, but 20 g of crude rivaroxaban (residual moisture content 10%) were used, the amounts of the other reagents adjusted accordingly and the time of mixing (at 110 to 115°C) was prolonged from 10 minutes to 2 hours. This provided a cream crystalline powder of rivaroxaban modification I in a yield of 94.0%.

In each case the purity of the rivaroxaban modification I obtained in Reference Examples 1-3 was assessed by HPLC according to the methodology used for Examples 1-9. The results are set out in Table 1.

**Table 1 - Overview of the crystallisation results (evaluation of purity of rivaroxaban according to the Eur. Specification)**

| | **Example No.** | | | | | | | | | | | | |
|---|---|---|---|---|---|---|---|---|---|---|---|---|---|
| | **1** | **2** | **3** | **4** | **5** | **6** | **7** | **8** | **9** | **10** | **R1** | **R2** | **R3** |
| **Reducing agent** | Na₂S₂O₄ | Na₂SO₃ | Na₂S | NaBH₄ | H₃PO₃ | L-ascorbic acid | D-(+)-glucose | dextrose | DMSO | Na₂S₂O₄ | - | - | - |
| **Appearance** | white | white | beige (light) | yellow (light) | white | white | beige (light) | beige (light) | white | white | white | pink/grey (light) | cream |
| **HPLC purity (%)** | 99.93 | 99.87 | 99.87 | 99.91 | 99.86 | 99.93 | 99.87 | 99.89 | 99.84 | 99.88 | 99.77 | 99.82 | 99.78 |
| **Yield (%)** | 83.3 | 82 | 83.3 | 87.3 | 83.7 | 84.7 | 83.3 | 78.7 | 86.7 | 85.3 | 93.1 | 94.2 | 94 |

## Claims

1. A process for the preparation of rivaroxaban modification I comprising:
(i) recrystallising rivaroxaban in acetic acid in the presence of a reducing agent; and
(ii) collecting the resultant rivaroxaban modification I.

2. A process for the preparation of rivaroxaban modification I comprising:
(i) treating acetic acid with a reducing agent;
(ii) separating the treated acetic acid from the reducing agent;
(iii) recrystallising rivaroxaban in the treated acetic acid; and
(iv) collecting the resultant rivaroxaban modification I.

3. The process as claimed in claim 1 or 2, wherein rivaroxaban modification I is **characterised by** XRPD peaks at 2 theta 16.5, 19.5, 22.5 and 23.4.

4. The process as claimed in any preceding claim, wherein the reducing agent is an alkali metal-containing reducing agent, a reducing sugar or a reducing acid agent.

5. The process as claimed in claim 4, wherein the alkali-metal containing reducing agent comprises sodium, lithium or potassium.

6. The process as claimed in claim 5, wherein the reducing agent is selected from sodium dithionate (Na₂S₂O₄), sodium sulfite (Na₂SO₃), sodium sulfide (Na₂S) or sodium borohydride (NaBH₄).

7. The process as claimed in claim 4, wherein the reducing agent is a reducing acid that is ascorbic acid.

8. The process as claimed in any preceding claim, wherein the reducing agent is used in an amount of from 1.0 mol% to 15 mol% relative to rivaroxaban, or from 2.0 mol% to 11.5 mol% relative to rivaroxaban, or from 5.0 mol% to 7.5 mol% relative to rivaroxaban.

9. The process as claimed in any preceding claim, wherein the recrystallisation step comprises combining rivaroxaban, acetic acid and the reducing agent and heating the resulting mixture to between 65°C and 95°C, or combining rivaroxaban and the treated acetic acid, and heating the resulting mixture to between 65°C and 95°C.

10. The process as claimed in claim 9, wherein the mixture is heated to between 70°C and 90°C, preferably between 80°C and 85°C.

11. The process as claimed in claim 9 or 10, wherein the mixture is heated and then stirred for a duration of between 2 to 6 hours, preferably 4 hours.

12. The process as claimed in any preceding claim, wherein rivaroxaban is prepared by reacting a compound of formula (I) or a salt thereof, with a compound of formula (II): 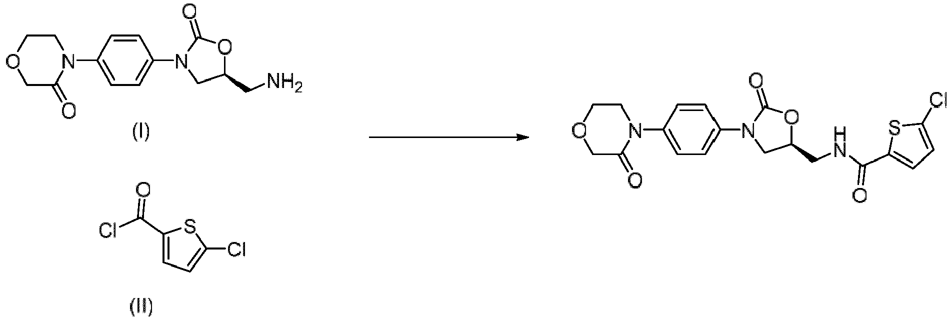 prior to the recrystallisation step.

13. The process as claimed in any preceding claim, wherein the rivaroxaban has not been subjected to column chromatography, or wherein the rivaroxaban is provided as a starting material for the preparation process as crude rivaroxaban having an impurity content of 0.10-10% by weight.

14. The process for preparing a rivaroxaban dosage form, comprising the steps of preparing rivaroxaban modification I as claimed in any preceding claim and combining the rivaroxaban modification I with one or more pharmaceutically acceptable excipients.

15. The process as claimed in claim 14, wherein the dosage form is an oral dosage form.
